# FASCICULE DE BREVET EUROPEEN

(11) **EP 1 625 152 B1**
(45) Date de publication et mention de la délivrance du brevet: **13.09.2006**
(21) Numéro de dépôt: 04742777.8
(22) Date de dépôt: 19.05.2004
(51) Int. Cl.: C07K 7/08, A61K 38/10

(54) **PEPTIDES MODULATEURS DE L'ACTIVITE DU FACTEUR DE TRANSCRIPTION ENGRAILED**
PEPTIDEN, DIE DIE AKTIVITÄT DES ENGRAILED-TRANSKRIPTIONSFAKTOR MODULIEREN
PEPTIDES MODULATING THE ACTIVITY OF THE ENGRAILED TRANSCRIPTION FACTOR

(30) Priorité: 20.05.2003 FR 0306023
(43) Date de publication de la demande: 15.02.2006
(73) Titulaire: CENTRE NATIONAL DE LA RECHERCHE SCIENTIFIQUE (CNRS), 75016 Paris (FR); Ecole Normale Superieure, 75230 Paris Cedex 05 (FR); Universite Paris 13, 93430 Villetaneuse (FR)
(72) Inventeur: PROCHIANTZ, Alain, F-75006 Paris (FR); LESAFFRE, Brigitte, F-93200 Saint-Denis (FR); VOLOVITCH, Michel, F-75005 Paris (FR); SONNIER, Laure, F-75002 Paris (FR)
(74) Mandataire: Vialle-Presles, Marie José
(86) Numéro de dépôt international: PCT/FR2004/001233
(87) Numéro de publication internationale: WO 2004/104030

(56) Documents cités:
- PELTENBURG L T C ET AL: "Engrailed and Hox homeodomain proteins contain a reated Pbx interaction motif that recognizes a common structure present in Pbx" 1996, THE EMBO JOURNAL, VOL. 15, NR. 13, PAGE(S) 3385-3393, 1996 , XP001154679
- DESPLAN C ET AL: "The Drosophila developmental gene, engrailed, encodes a sequence-specific DNA binding activity" 1985, NATURE, VOL. 318, PAGE(S) 630-635, 1985 , XP001154680

## Description

L'invention est relative à des peptides capables de modifier l'activité de facteurs de transcription " Engrailed ".

Les protéines Engrailed sont des facteurs de transcription de la classe des protéines à homéodomaine. Les mammifères possèdent deux homologues d'Engrailed : Engrailed-1 et Engrailed-2 ; ces deux protéines qui ont une activité similaire, seront collectivement désignées ci-après sous le terme général d'Engrailed (EN).

Chez le nouveau né et chez l'adulte, EN est exprimé dans les neurones dopaminergiques (DA) de la substance noire (qui dégénèrent dans la maladie de Parkinson) et les noyaux du raphé et du locus coeruleus qui jouent un rôle important dans la régulation de l'humeur et la mise en place des comportements addictifs.

Il a été montré (SIMON et al. J Neurosci. 21 (9):3126-34, 2001) que la perte de EN au cours du développement est suivie assez rapidement par la dégénérescence des neurones dopaminergiques, et que l'une des cibles transcriptionnelles de EN est l'alphasynucléine, dont la liaison génétique avec certaines formes familiales de la maladie de Parkinson a été montrée (POLYMEROPOULOS et al., Science. 276, 2045-7, 1997), et qui constituerait un régulateur négatif de la transmission dopaminergique (ABELIOVICH et al., Neuron, 25, 239-52, 2000).

L'ensemble de ces observations suggère fortement l'implication d'Engrailed dans des pathologies neurodégénératives, et notamment dans des pathologies affectant les neurones dopaminergiques, telles que la maladie de Parkinson. Son expression dans les neurones du raphé et du locus coeruleus soulève l'hypothèse d'une implication dans d'autres pathologies, comme les troubles de l'humeur ou l'addiction.

Il apparaît donc particulièrement souhaitable de disposer de modulateurs de l'activité d'Engrailed.

Les Inventeurs ont recherché s'il existait des peptides capables de moduler l'activité d'Engrailed par fixation à la protéine. Ils ont ainsi identifié deux peptides qui se fixent sur Engrailed, et peuvent moduler de manière spécifique son activité, *in vitro*, aussi bien qu' *in vivo.*

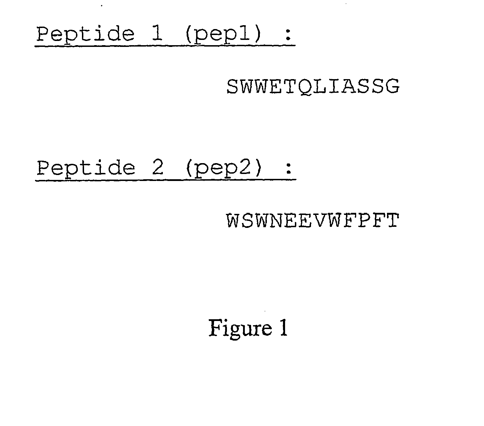

Ces peptides : SWWETQLIASSG (Peptide 1 ; SEQ ID NO : 1) et WSWNEEVWFPFT (Peptide 2 ; SEQ ID NO : 2) sont représentés sur la figure 1.

Dans des cellules transformées surexprimant Engrailed, le peptide 1 a un effet activateur, alors que le peptide 2 a un effet inhibiteur. Dans un contexte physiologique, les deux peptides ont un effet activateur. En outre les Inventeurs ont constaté que ces peptides conféraient des propriétés d'activation de la transcription à l'homéodomaine d'Engrailed, qui, en lui-même, ne possède pas ces propriétés.

La présente invention a pour objet un peptide capable de se fixer au facteur de transcription Engrailed, et de réguler l'activité dudit facteur de transcription, choisi parmi les peptides de séquence SWWETQLIASSG et WSWNEEVWFPFT.

La présente invention a également pour objet l'utilisation d'un peptide conforme à l'invention pour réguler in vitro l'activité du facteur de transcription Engrailed dans une cellule vivante.

Pour la mise en oeuvre de la présente invention, ledit peptide peut être introduit dans la cellule de différentes manières.

Avantageusement, il peut être associé à un peptide comprenant un domaine transducteur.

On désigne sous le terme de" domaine transducteur " une séquence peptidique capable de pénétrer à l'intérieur d'une cellule vivante, indépendamment de la présence de transporteurs ou de récepteurs spécifiques, et capable d'importer dans ladite cellule des molécules ou complexes moléculaires de nature variée (acides nucléiques, protéines, peptides/acides nucléiques, analogues de nucléotides, liposomes), habituellement désignés sous le terme général de " cargos ".

Des peptides transducteurs de différents types sont connus en eux-mêmes. Pour revue cf. par exemple LIDGREN *et al.,* TiPS, 21, 99-102, (2000) ; SCHWARZE et DOWDY TiPS, 21, 45-48, (2000) ; SCHWARZE *et al.* Trends Cell. Biol., 10, 290-295, (2000) ; PROCHIANTZ Current Opinion in Cell Biology, 12, 400-406, (2000) ; Cell-Penetrating Peptides. Processes and applications. Ed. Ulo Langel. CRC Press (2002).

Selon un mode de mise en oeuvre préféré de la présente invention, on peut associer un peptide régulateur conforme à l'invention à un peptide comprenant un domaine transducteur de type pénétratine, dérivé de la troisième hélice d'un homéodomaine ; des peptides de la famille des pénétratines sont décrits par exemple dans les publications de JOLIOT et al. Proc. Natl. Acad. Sci. USA, 88, 1864-1868, (1991) ; DEROSSI *et al.* J. Biol. Chem., 269, 14, 10444-10450, (1994) ; BRUGIDOU et al. Biophys. Biochem. Res. Com., 214, 685-693, (1995), ainsi que dans le Brevet US 5888762, le Brevet US 6080724, ou la Demande PCT WO 00/01417 ou la Demande PCT WO 00/29427.

La présente invention a également pour objet une composition comprenant un peptide régulateur conforme à l'invention associé à un peptide comprenant un domaine transducteur, de préférence un domaine transducteur de type pénétratine.

Selon un mode de réalisation préféré d'une composition conforme à l'invention, le peptide régulateur conforme à l'invention est associé à Engrailed, ou à un fragment d'Engrailed comprenant au moins son homéodomaine.

L'association entre ces peptides peut s'effectuer par liaison covalente, comme décrit dans les documents mentionnés ci-dessus, ou bien par liaison non-covalente, comme décrit dans la Demande FR 03/00093.

La présente invention englobe aussi tout polypeptide chimérique comprenant un peptide conforme à l'invention fusionné à un peptide hétérologue. Selon un mode de réalisation particulier de la présente invention, ledit peptide hétérologue comprend au moins un domaine transducteur, de préférence un domaine transducteur de type pénétratine. Selon un mode de réalisation préféré d'un polypeptide chimérique conforme à l'invention, il s'agit d'un facteur de transcription chimérique, comprenant un peptide conforme à l'invention fusionné à un fragment d'Engrailed comprenant au moins son homéodomaine.

La présente invention a également pour objet un polynucléotide choisi parmi :
- un polynucléotide codant pour un peptide régulateur conforme à l'invention ;
- un polynucléotide codant pour un polypeptide chimérique conforme à l'invention.

La présente invention englobe également un vecteur recombinant comprenant un polynucléotide conforme à l'invention.

La présente invention englobe aussi une composition comprenant un polynucléotide codant pour un peptide régulateur conforme à l'invention et un polynucléotide codant pour un peptide hétérologue, notamment un peptide comprenant un domaine transducteur, de préférence un domaine transducteur de type pénétratine. Une composition conforme à l'invention peut par exemple comprendre un vecteur recombinant comprenant une séquence codant pour un peptide régulateur conforme à l'invention, et une séquence codant pour ledit peptide hétérologue, les deux séquences pouvant être ou non adjacentes. Alternativement, une composition conforme à l'invention peut comprendre deux vecteurs différents, l'un portant une séquence codant pour un peptide régulateur conforme à l'invention, et l'autre portant une séquence codant pour le peptide hétérologue.

Des vecteurs comprenant un polynucléotide conforme à l'invention peuvent être utilisés, si on le souhaite, pour introduire et exprimer ce polynucléotide dans la cellule dans laquelle on souhaite réguler l'expression d'Engrailed.

On peut employer dans ce but les vecteurs habituellement utilisés pour exprimer un gène d'intérêt dans des cellules animales, par exemple des vecteurs viraux. Avantageusement, on peut utiliser comme vecteur un bactériophage sur lequel est adsorbé un peptide transducteur, tel que ceux décrits dans la Demande FR 03/00093.

La présente invention a également pour objet l'utilisation d'un peptide, d'un polynucléotide, ou d'une composition tel que définis ci-dessus, pour l'obtention d'un médicament, utilisable notamment dans le cadre du traitement de pathologies du système nerveux, par exemple des pathologies neurodégénératives, telles que la maladie de Parkinson.

La présente invention sera mieux comprise à l'aide du complément de description qui va suivre, qui se réfère à des exemples non-limitatifs illustrant l'obtention de peptides capables de se fixer à Engrailed, et de moduler son activité.

### EXEMPLE 1: OBTENTION DE PEPTIDES CAPABLES DE MODULER L'ACTIVITE D'ENGRAILED

### 1) Système de test de l'activité d'Engrailed

L'activité transcriptionnelle d'Engrailed est évaluée comme décrit par MONTESINOS et al. (J. Neurosci., 21, 3350-9, 2001), par mesure de l'expression du gène rapporteur luciférase placé dans le plasmide pMAP-luc sous contrôle d'un promoteur cible d'Engrailed, le promoteur *MAP1B* (microtubule-associated protein 1B).

Des cellules de la lignée neuroépithéliale CHP 100 (SCHLESINGER et al, Cancer Res. 36 , 3904-3100, 1976) sont mises en culture dans du RPMI 1640 (Life Technologies, Gaithersburg, MD) additionné de 16mM glucose, 15% sérum de veau foetal, 5U/ml de pénicilline et 5µ g/ml de streptomycine, et co-transfectées par électroporation avec pMAP-luc et l'un des vecteurs suivants :
**pCL9mEn2** (MAINGUY et al., Nat Biotechnol. *18 :* 746-749, 2000) qui contient la séquence codant pour la protéine Engrailed-2 de poulet entière, sous contrôle du promoteur CMV ;
**pCL9mEn2ΔH1** (JOLIOT et al., Curr Biol. *8* : 856-863, 1998), qui contient la séquence codant pour la protéine Engrailed-2 de poulet délétée des acides aminés 36 à 46 de l'homéodomaine (séquence permettant la liaison à l'ADN), sous contrôle du promoteur CMV ;
**pCL9mHDEn2C**, qui contient la séquence codant pour la protéine Engrailed-2 de poulet délétée des acides aminés 10 à 185, sous contrôle du promoteur CMV ;

L'électroporation est effectuée avec 10 pg d'ADN plasmidique au maximum (2 µg plasmide rapporteur pMAP-luc + quantités variables de vecteur d'expression de En2) de pour 8 x 10⁵ cellules dans 350 µl de milieu, à 1050 pF et 260 mV. On ajoute ensuite 400 µl de milieu et on laisse reposer 10 min. Les cellules sont alors mises en culture, après un lavage, dans deux boîtes (3,5 cm diamètre).

L'activité luciférase est dosée 24 heures après transfection. Les cellules sont rincées avec du PBS. Du tampon de lyse (Tris/H₃P0₄ pH 7,8 20mM, MgCl₂ 10mM, glycérol 15%, EDTA 1mM, Triton-X 100 1%, ATP 1,25mM, luciférine 10µg/ml) est ajouté aux cellules. Après incubation 30min à 4°C, la mesure de l'activité luciférase est effectuée par luminométrie(luminomètre Lumat LB 9501 Berthold).

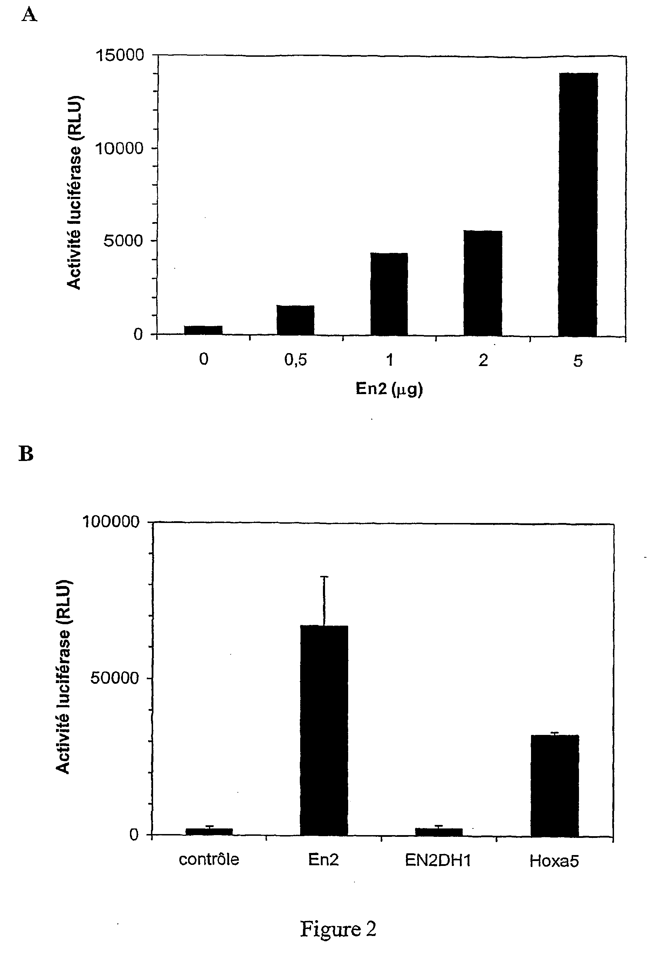

Les résultats sont illustrés par les Figures 2A et 2B.

La Figure 2A montre que la protéine Engrailed-2 (En2) est capable d'activer l'expression du promoteur *MAP1B* de manière dose dépendante. La Figure 2B montre que cette activation nécessite la liaison de l'homéodomaine au promoteur. Celui-ci n'est en effet pas activé par la protéine recombinante EN2ΔH1.

Le promoteur *MAP1B* est aussi activé par une autre protéine à homéodomaine, HOXA5.

### 2) Sélection de peptides capables de se fixer à Engrailed.

Des peptides se liant spécifiquement à Engrailed ont été recherchés par criblage d'une banque de phages (PhD-12, New England Biolabs) exprimant à leur surface des peptides aléatoires de 12 acides aminés. Plusieurs peptides capables de se fixer sur Engrailed ont ainsi été obtenus.

Les deux peptides les plus représentés sont SWWETQLIASSG (Pep1; SEQ ID NO: 1) et WSWNEEVWFPFT (Pep2 ; SEQ ID NO: 2). Ces peptides ont été choisis pour tester leur effet sur l'activité transcriptionnelle d'Engrailed. Les séquences de ces peptides sont illustrées par la figure 1.

Les séquences codant pour Pep1 ou Pep2 ont été placées dans le vecteur pCS2+ (TURNER et WEINTRAUB, Genes and Development. *8* : 1311-1323, 1994), pour donner respectivement les vecteurs d'expression pCS2-pep1 et pCS2-pep2.

### 3)Test de l'effet des peptides sélectionnés sur l'activité d'Engrailed dans des cellules CHP100.

Les cellules CHP100 sont co-transfectées par électroporation, comme décrit ci-dessus, avec pMAP-luc (2 µg), pCL9mEn2 ou pCL9mHDEn2C (4µg) et 4µg de pCS2-pep1 ou pCS2-pep2.

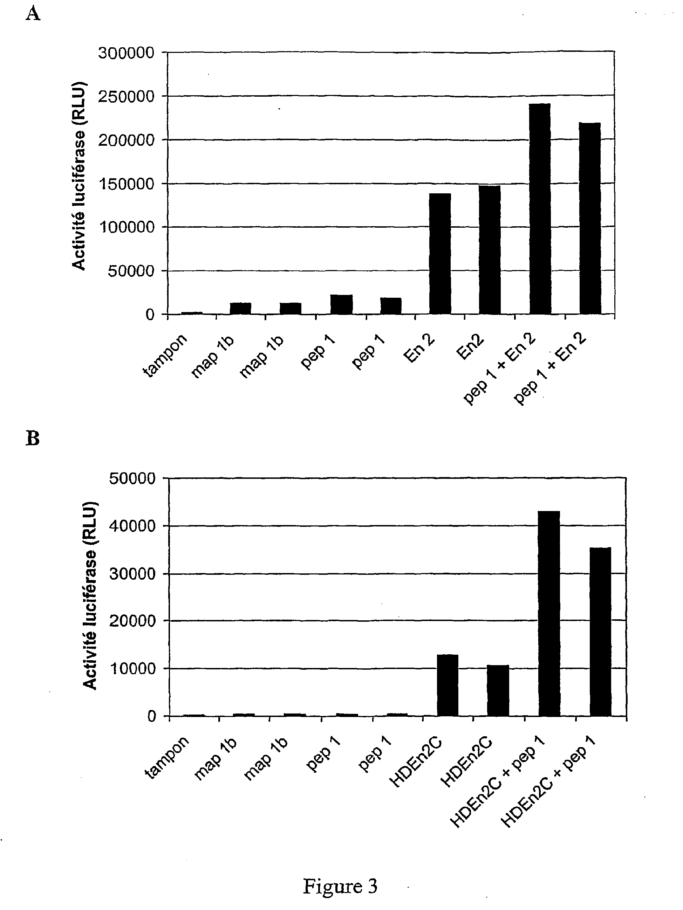

La Figure 3A montre que lorsque le peptide 1 est introduit dans les cellules en même temps qu'Engrailed (pep 1+En2) l'activation de *MAP1B* est augmentée d'environ 50%. Le peptide seul n'a pas d'action sur ce promoteur (pepl). Le promoteur *MAP1B* n'est pas actif par lui-même dans ce type cellulaire (map1b).

La Figure 3B montre que le peptide HDEn2C (constitué principalement par l'homéodomaine d'Engrailed) n'active que faiblement le promoteur *MAP1B* (HDEn2C), et que cette activation est fortement augmentée par le peptide 1 (HDEn2C+pep1).

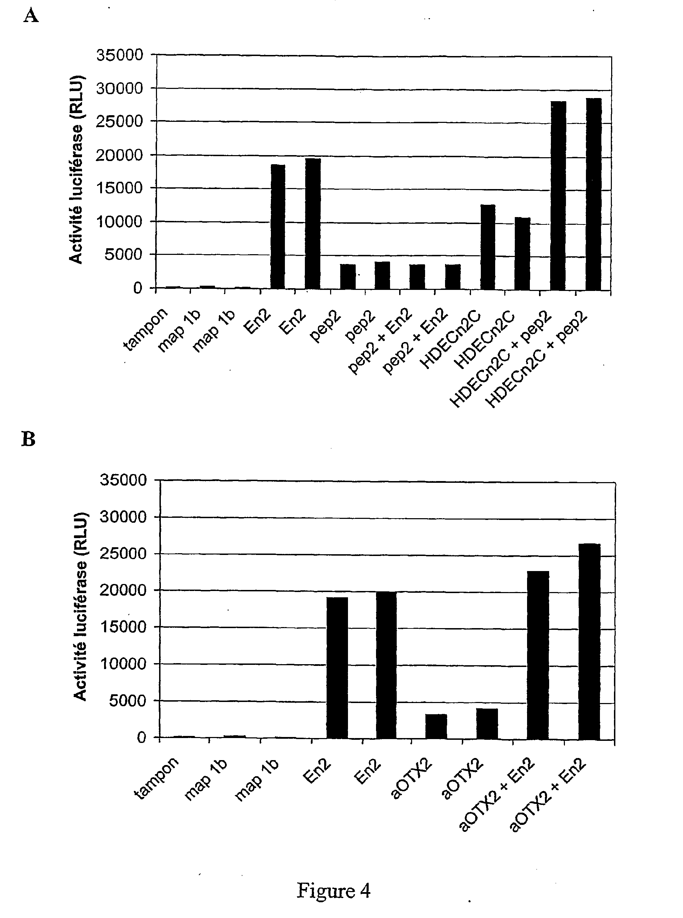

La Figure 4A montre que le peptide 2, qui n' a pas d'effet par lui-même sur *MAP1B* (pep2), est inhibiteur de l'activation de *MAP1B* par Engrailed (pep2+En2), et au contraire, augmente l'activation de *MAP1B* par le peptide HDEn2C (HDEn2C+pep2).

La Figure 4B montre qu'un peptide contrôle (aOTX2) de séquence KVWDIRYTTPHA (SEQ ID NO :3) et se fixant spécifiquement à l'homéoprotéine OTX2 ne modifie pas l'activation de *MAP1B* par Engrailed (aOTX2+En2). L'effet du peptide aOTX2 et celui de Engrailed sont purement additifs. **4) Test de l'effet des peptides sélectionnés sur l'activité d'Engrailed dans des cultures primaires de neurones de mésencéphale.**

Afin de vérifier la fonctionnalité des peptides 1 et 2 dans un contexte cellulaire physiologique, l'effet de ces peptides a été testé dans des cultures primaires de neurones de mésencéphale d'embryon de souris.

Des fragments de mésencéphale de souris (jour embryonnaire 13,5) sont incubés 5 min. à 24°C dans de la trypsine-EDTA, puis lavés dans un tampon phosphate pH7,5 additionné de 33mM glucose (PBS) et 10% sérum de veau foetal, et ensuite incubés 10min. à 37°C dans 30µM/ml de DnaseI (Sigma, Saint Louis, MO). Les cellules sont dissociées mécaniquement, lavées trois fois au PBS et mises en culture à la densité de 200000 cellules/cm², dans des puits préalablement saturés par de la D,L-polyornithine (1,5µg/ml) et de la laminine (5µg/ml). Le milieu de culture (MMS) est constitué de DMEM/F12 (1/1, Life Technologies, Cergy, France), avec 33mM glucose, 2mM glutamine, 10mM HEPES, pH7,4, 9mM NaHCO⁻₃, 5U/ml pénicilline et 5µg/ml streptomycine. Au MSS sont additionnés 0,1% d'ovalbumine, 25µg/ml d'insuline, 100µg/ml de transferrine, 20nM de progestérone, 60µM putrescine et 30nM sélénium (M20V).

Le lendemain de la mise en culture, dans chaque puits, le milieu est remplacé par 600µl de MSS, sans pénicilline ni streptomycine. On ajoute à chaque puits 100µl du mélange de transfection comprenant pMAP-luc (0,5µg) et 0,5µg de pCS2-pep1 ou pCS2-pep2, et 1mg/ml de LIPOFECTAMINE 2000 (Invitrogen) dans du milieu OPTI-MEM (qsp 100µl), préalablement mélangés pendant 20min à 24°C. Les cellules sont alors incubées 2h30 à 37°C. Ensuite le milieu est remplacé par du M20V.

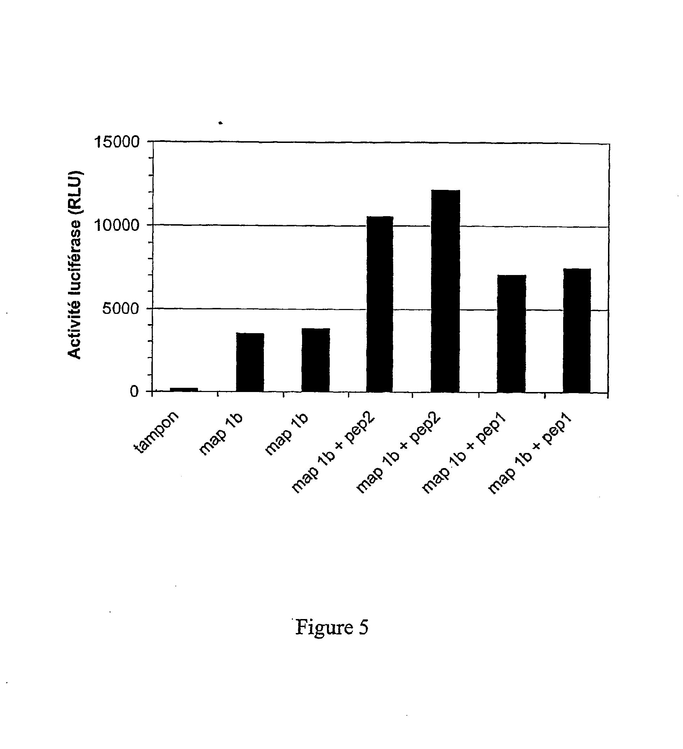

Les résultats sont illustrés par la Figure 5.

Ces résultats montrent que le promoteur *MAP1B* est activé dans ces neurones (map1b), et que cette activation est augmentée par le peptide 1 (map1b+pep1) ainsi que par le peptide 2 (map1b+pep2), l'effet activateur du peptide 2 apparaissant, dans ce contexte, plus important que celui du peptide 1.

### EXEMPLE 2 : EFFET IN VIVO DES PEPTIDES PEP-1 ET PEP-2

L'effet de pep1 ou pep2 sur l'activité transcriptionnelle d'Engrailed a été évalué par mesure de l'expression du gène rapporteur β-galactosidase placé dans le plasmide pMAP-lacZ (décrit par MONTESINOS et al., 2001, précité) sous contrôle du promoteur *MAP1B* dans le tube neural d'embryons de poulet au stade HH8-HH10.

Les vecteurs pCS2-pep1 ou pCS2-pep2, ainsi que le vecteur pMAP-lacZ (chaque vecteur est utilisé à une concentration de 1µg/µl) ont été introduits par électroporation dans le tube neural d'embryons de poulet au stade HH8-HH10 conformément au protocole de MURAMATSU et al. (1997), avec un électroporateur BTX, ECM 830 (4 pulses de 25V et 50msec) (Genetronics, San Diego, CA). Les vecteurs ont été injectés dans le tube neural grâce à une micropipette. Après 24h d'incubation à 37°C, les embryons ont été fixés et l'activité β-galactosidase révélée, comme décrit par HOGAN et al, (Manipulating the mouse embryo. A laboratory manual. Cold Spring Harbor, NY : Cold Spring Harbor laboratory, 1994).

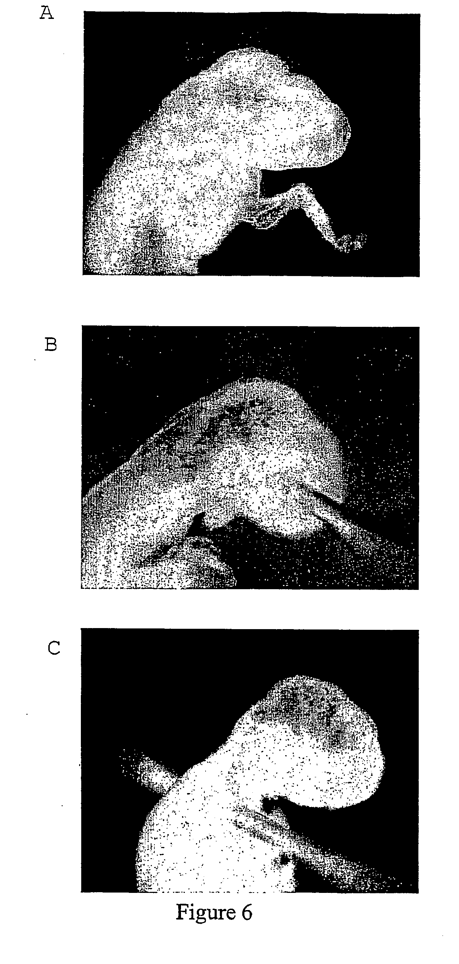

Les résultats sont illustrés par la Figure 6 (A-C):

La figure 6A (pMap-LacZ) montre que le promoteur *MAP1B* est activé *in vivo* dans la région mesmétencéphalique correspondant à l'expression normale de Engrailed. Cette expression est confinée aux régions ventrales qui seules sont accessibles aux plasmides du fait de la technique d'électroporation dans le tube nerveux.

La figure 6B (pMap-LacZ + pCS2-Pep1) montre que cette activation est augmentée par le peptide 1. Le marquage semble aussi plus étendu.

La Figure 6C (pMap-LacZ + pCS2-Pep2) montre que le peptide 2 augmente aussi l'activation de *MAP1B.*

## Revendications

1. Peptide capable de se fixer au facteur de transcription Engrailed, choisi parmi :
- le peptide de séquence SWWETQLIASSG ;
- le peptide de séquence WSWNEEVWFPFT.

2. Composition comprenant un peptide selon la revendication 1, associé à un peptide comprenant un domaine transducteur.

3. Composition selon la revendication 2, **caractérisée en ce que** ledit peptide transducteur est une pénétratine.

4. Composition selon la revendication 3, **caractérisée en ce que** ledit peptide transducteur est la protéine Engrailed, ou un fragment de celle-ci comprenant au moins son homéodomaine.

5. Composition selon une quelconque des revendications 2 à 4, **caractérisée en ce qu'**elle est constituée par un polypeptide chimérique.

6. Polynucléotide choisi parmi :
- un polynucléotide codant pour un peptide selon la revendication 1 ;
- un polynucléotide codant pour un polypeptide chimérique tel que défini dans la revendication 5.

7. Utilisation d'un peptide selon la revendication 1, d'une composition selon une quelconque des revendications 2 à 5, ou d'un polynucléotide selon la revendication 6 pour réguler *in vitro* l'activité du facteur de transcription Engrailed dans une cellule vivante.

8. Peptide selon la revendication 1, composition selon une quelconque des revendications 2 à 5, ou polynucléotide selon la revendication 6, pour utilisation comme médicament.

## Claims

1. Peptide capable of fixing itself to the Engrailed transcription factor, chosen from:
- the peptide of sequence SWWETQLIASSG
- the peptide of sequence WSWNEEVWFPFT.

2. Composition comprising a peptide according to claim 1, associated with a peptide comprising a transducing domain.

3. Composition according to claim 2, **characterised in that** said transducing peptide is a penetratin.

4. Composition according to claim 3, **characterised in that** said transducing peptide is the Engrailed protein, or a fragment thereof comprising at least its homeodomain.

5. Composition according to any one of claims 2 to 4, **characterised in that** it is constituted by a chimeric polypeptide.

6. Polynucleotide chosen from:
- a polynucleotide encoding a peptide according to claim 1;
- a polynucleotide encoding a chimeric polypeptide as defined in claim 5.

7. Use of a peptide according to claim 1, of a composition according to any one of claims 2 to 5, or of a polynucleotide according to claim 6 for regulating *in vitro* the activity of the Engrailed transcription factor in a living cell.

8. Peptide according to claim 1, composition according to any one of claims 2 to 5, or polynucleotide according to claim 6, for use as a medicament.

## Patentansprüche

1. Peptid, das fähig ist, an den Engrailed-Transkriptionsfaktor zu binden, ausgewählt aus:
- dem Peptid der Sequenz SWWETQLIASSG;
- dem Peptid der Sequenz WSWNEEVWFPFT.

2. Zusammensetzung, umfassend ein Peptid nach Anspruch 1, assoziiert mit einem Peptid, das eine transduzierende Domäne umfasst.

3. Zusammensetzung nach Anspruch 2, **dadurch gekennzeichnet , dass** das transduzierende Peptid ein Penetratin ist.

4. Zusammensetzung nach Anspruch 3, **dadurch gekennzeichnet , dass** das transduzierende Peptid das Engrailed-Protein oder ein Fragment dieses, das wenigstens seine Homeodomäne umfasst, ist.

5. Zusammensetzung nach einem der Ansprüche 2 bis 4, **dadurch gekennzeichnet , dass** sie durch ein chimäres Polypeptid gebildet wird.

6. Polynucleotid, ausgewählt aus:
- einem Polynucleotid, das für ein Peptid nach Anspruch 1 codiert;
- einem Polynucleotid, das für ein chimäres Polypeptid, wie es in Anspruch 5 definiert ist, codiert.

7. Verwendung eines Peptids nach Anspruch 1, einer Zusammensetzung nach einem der Ansprüche 2 bis 5 oder eines Polynucleotids nach Anspruch 6 zur Regulierung der *in vitro*-Aktivität des Engrailed-Transkriptionsfaktors in einer lebenden Zelle.

8. Peptid nach Anspruch 1, Zusammensetzung nach einem der Ansprüche 2 bis 5 oder Polynucleotid nach Anspruch 6 zur Verwendung als Medikament.


### SEQUENCE LISTING

<110> CENTRE NATIONAL DE LA RECHERCHE SCIENTIFIQUE
   ECOLE NORMALE SUPERIEURE
   UNIVERSITE PARIS 13
   PROCHIANTZ, Alain
   LESAFFRE, Brigitte
   VOLOVITCH, Michel
   SONNIER, Laure
<120> PEPTIDES MODULATEURS DE L'ACTIVITE DU FACTEUR DE TRANSCRIPTION ENGRAILED
<130> MJPbv644/101
<150> 03 06023
   <151> 2003-05-20
<160> 3
<170> PatentIn version 3.1
<210> 1
   <211> 12
   <212> PRT
   <2I3> Artificial sequence
<220>
   <223> peptide
<400> 1
   Ser Trp Trp Glu Thr Gln Leu Ile Ala Ser Ser Gly
   1               5                   10
<210> 2
   <211> 12
   <212> PRT
   <213> Artificial sequence
<220>
   <223> peptide
<400> 2
   Trp Ser Trp Asn Glu Glu Val Trp Phe Pro Phe Thr
   1               5                   10
<210> 3
   <211> 12
   <212> PRT
   <213> Artificial sequence
<220>
   <223> peptide
<400> 3
   Lys Val Trp Asp Ile Arg Tyr Thr Thr Pro His Ala
   1               5                   10